# EUROPEAN PATENT SPECIFICATION

(11) **EP 4 021 319 B1**
(45) Date of publication and mention of the grant of the patent: **06.12.2023**
(21) Application number: 20768515.7
(22) Date of filing: 25.08.2020
(51) Int. Cl.: A61B 17/34, A61M 37/00, A61B 90/00

(54) **INJECTOR SYSTEM FOR DELIVERY OF A MEDICAL IMPLANT**
INJEKTORSYSTEM ZUR EINFÜHRUNG EINES MEDIZINISCHEN IMPLANTATS
SYSTÈME D'INJECTEUR D'UN MARQUEUR DE REPÈRE

(30) Priority: 26.08.2019 BE 201905555
(43) Date of publication of application: 06.07.2022
(73) Proprietor: Novalon S.A., 4000 Liège (BE)
(72) Inventor: BUFFAT, Jean-Michel, 69008 Lyon (FR); VAN BUTSELE, Kathy, 4053 Embourg (BE)
(74) Representative: De Clercq & Partners
(86) International application number: PCT/EP2020/073764
(87) International publication number: WO 2021/037860

(56) References cited:
- EP-A1- 0 639 387
- US-A- 5 201 779
- US-A- 5 772 671
- US-A1- 2002 188 247
- US-A1- 2009 270 797
- US-A1- 2010 331 874

## Description

### Field of the invention

The present invention is in the field of injector systems for medical implants, in particular for drug-eluting medical implants.

### Background to the invention

The injection of a medical implant is typically via a subcutaneous, intramuscular, or intradermal injection route and entails advancing an implant loaded into a syringe along a bore of an injection needle into tissue of the patient. The implant may be any, for instance, a drug-eluting implant, a radio-opaque marker, encapsulated electronic device. Where the implant is drug-eluting, it typically elutes one or more active substances for a prolonged period, for instance, for the delivery of an LHRH agonist or antagonist (e.g. goserelin, leuprorelin or buserelin) for the treatment of hormone sensitive cancers such as breast or prostate cancers or for treatment of benign gynaecological disorders (e.g. endometriosis, uterine fibroids and endometrial thinning).

The diameter of the implant needs to be carefully controlled; it governs the forces required for injection. Regulating the diameter of the implant is paramount to proper storage and transport also. If the implant is greater than a specified dimensional tolerance, injection forces can be excessive and it can lead to damage of the implant. If the implant is smaller than a specified dimensional tolerance, the implant can drop out from a pre-loaded syringe during transport or during preparation by the administering physician. At the same time, it is desirable to minimise the diameter of the needle used to inject the implant. A larger diameter needle causes more pain to the subject, which in some cases requires a local anaesthetic; avoidance of administrating additional medication (e.g. opiates) is desirable. Further, a larger needle diameter increases trauma and healing time. In addition, an ability to easily adapt an injector to administer implant of different diameters and doses would lead to significant cost saving in manufacturing and seeking regulatory approval.

In US 5,772,671, the implant is damaged by a plurality friction ribs that prevent the implant from falling out, but which damage the implant as it passes through (see **FIGs. 7A** to **10B** herein). In US 5,201,779, the device is for injection of a gel-like silicone implant through a nozzle and into a surgical incision; such a device is incompatible with a needle for puncture of an organ (e.g. skin). In US 2002/0188247, EP 0 639 387, US 2010/0331874 a large diameter of needle is employed in order to accommodate a retaining spring mechanism within or as part of the needle itself, thereby increasing pain, trauma and healing time. In US 2009/0270797 a spring mechanism is used to retain a needle within a body of an injector, however, the implant falls out once the needle is advanced past the retaining mechanism.

It is an aim of the present invention to provide an injector system that overcomes the problems of the art, and which maximises the range of implants deliverable by an injector system.

### Summary of the invention

The invention is defined in claim 1. Further embodiments are defined in the dependent claims.

Described herein is an injector system (500) for injection by puncture of an organ, of a non-deformable medical implant (200) into a subject comprising:
- a holding element (100) having a proximal (10) and distal (20) end disposed with a chamber (102) for containing the implant (200),
- wherein the holding element (100) is disposed with an exit port (104) at the distal end (20) for slidable ejection of the implant (200) therethrough and into a bore (404) of a needle component (402) of an injection needle assembly (400),
- a compliant member assembly (150) having a through-passage (154), comprising at least two compliant members (152), the compliant member assembly (150) having a biased state in which the at least two compliant members (152) additively form a partial or total occlusion of the through-passage (154), wherein:
   - the compliant member assembly (150) is configured in the biased state as a mechanical stop adapted to stop slidable entry of the implant (200) into the bore (404) under a force of gravity,
   - the through-passage (154) is configured to receive slidably the implant under a force of injection in which the at least two compliant members (152) deform and frictionally engage the implant (200),
   - the complaint member assembly (150) is configured to align the implant with the bore of the needle component

Described herein is an injector system (500) for injection of a medical implant (200) into a subject comprising:
- a holding element (100) having a proximal (10) and distal (20) end disposed with a chamber (102) for containing the implant (200),
- wherein the holding element (100) is disposed with an exit port (104) at the distal end (20) for slidable ejection of the implant (200) therethrough,
- a compliant member assembly (150) comprising at least one compliant member (152) configured to frictionally engage the implant (200) to impede passage therethrough, and
- wherein the implant (200) is slidable relative to the compliant member assembly (150).

The injector system (500) may further comprising an injection needle assembly (400) in fluid connection with the exit port (104) of the holding element (100), which needle assembly (400) comprises a needle component (402) disposed with a bore (404) for the passage of the implant (200) wherein the compliant member assembly (150) is configured to align the implant (200) for passage through the bore (404). The number of compliant members (152) may be 2 or more. The compliant member assembly (150) may comprise 2, 3 or 4 compliant members (152) arranged in the compliant member assembly (150) to form an essentially circular profile. The compliant member assembly (150) may comprise an aperture defined by the compliant members (152), preferably by inward pointing edges of the compliant members (152), the aperture having a minimum width smaller than a minimum width of the implant (200) transverse profile. The compliant member assembly (150) may be biased in an essentially planar configuration. The compliant member assembly (150) may be disposed within the holding element (100), or, when the injection needle assembly (400) is present, within the needle assembly (400). The compliant member assembly (150) may be further configured as a compliant mechanical stop adapted to stop slidable entry of the implant (200) into the compliant member assembly (150) and through the exit port (104) under a force of gravity. The injector system (500) may be configured such that frictional engagement locks the position of the implant (200) relative to the compliant member assembly (150), wherein an injection force applied to the implant (200), preferably of less than 10N, overcomes the lock. The injector system (500) may further comprising a syringe (300) having a syringe barrel (320), wherein the holding element (100) is disposed within the syringe barrel (320). The holding element (100) may be a syringe barrel (320). The injector system (500) may further comprise the implant (200). The injector system (500) may be configured for subcutaneous injection, intramuscular or intradermal injection of the implant (200). The injector system (500) may further comprising a needle protection mechanism.

### Figure Legends

**FIG. 1A** is a cross-section view of an injector system of the invention where the compliant member stops passage of the implant under the force of gravity, thereby retaining the implant within the chamber of the holding element.
**FIG. 1B** is a cross-section view of an injector system of the invention where the compliant member assembly engages with the implant to impede its passage through the compliant member assembly, thereby retaining the implant within the chamber of the holding element.
**FIG. 2** shows a cross-sectional view of an injector system of the invention, wherein the holding element is disposed within a barrel of a syringe.
**FIG. 3** shows a cross-sectional view of an injector system of the invention, wherein the holding element is a barrel of a syringe.
**FIG. 4****.** depicts an injector system of the invention comprising a holding element and a needle assembly, wherein the compliant member stops is part of the needle assembly.
**FIGs. 5** and **6** depict possible configurations of compliant members assemblies.
**FIG. 7A** is a photograph of an arrangement of rigid ribs each having a round profile; **FIG. 7B** is a schematic view of the configuration.
**FIG. 8A** is a photograph of an arrangement of rigid ribs each having a triangular profile;
**FIG. 8B** is a schematic view of the configuration.
**FIG. 9A** is a photograph of an arrangement of rigid ribs each having a rectangular profile;
**FIG. 9B** is a schematic view of the configuration.
**FIG. 10A** is a photograph of an arrangement of rigid ribs each having a triangular profile;
**FIG. 10B** is a schematic view of the configuration.
**FIG. 11A** is a photograph of a compliant member assembly according to the invention;
**FIG. 11B** is a schematic view of the configuration.

### Detailed description of invention

Before the present system and method of the invention are described, it is to be understood that this invention is not limited to particular systems and methods or combinations described, since such systems and methods and combinations may, of course, vary. It is also to be understood that the terminology used herein is not intended to be limiting, since the scope of the present invention will be limited only by the appended claims.

As used herein, the singular forms "a", "an", and "the" include both singular and plural referents unless the context clearly dictates otherwise.

The terms "comprising", "comprises" and "comprised of" as used herein are synonymous with "including", "includes" or "containing", "contains", and are inclusive or open-ended and do not exclude additional, non-recited members, elements or method steps. It will be appreciated that the terms "comprising", "comprises" and "comprised of" as used herein comprise the terms "consisting of', "consists" and "consists of".

The recitation of numerical ranges by endpoints includes all numbers and fractions subsumed within the respective ranges, as well as the recited endpoints.

The term "about" or "approximately" as used herein when referring to a measurable value such as a parameter, an amount, a temporal duration, and the like, is meant to encompass variations of +/-10% or less, preferably +/-5% or less, more preferably +/-1% or less, and still more preferably +/-0.1 % or less of and from the specified value, insofar such variations are appropriate to perform in the disclosed invention. It is to be understood that the value to which the modifier "about" or "approximately" refers is itself also specifically, and preferably, disclosed.

Whereas the terms "one or more" or "at least one", such as one or more or at least one member(s) of a group of members, is clear *per se*, by means of further exemplification, the term encompasses *inter alia* a reference to any one of said members, or to any two or more of said members, such as, e.g., any ≥3, ≥4, ≥5, ≥6 or ≥7 etc. of said members, and up to all said members.

All references cited in the present specification are hereby incorporated by reference in their entirety. In particular, the teachings of all references herein specifically referred to are incorporated by reference.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

In the following passages, different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the invention, and form different embodiments, as would be understood by those in the art. For example, in the appended claims, any of the claimed embodiments can be used in any combination.

In the present description of the invention, reference is made to the accompanying drawings that form a part hereof, and in which are shown by way of illustration only of specific embodiments in which the invention may be practiced. Parenthesized or emboldened reference numerals affixed to respective elements merely exemplify the elements by way of example, with which it is not intended to limit the respective elements. It is to be understood that other embodiments may be utilised and structural or logical changes may be made without departing from the scope of the present invention. The following detailed description, therefore, is not to be taken in a limiting sense, and the scope of the present invention is defined by the appended claims.

The terms "distal" and "proximal" are used through the specification, and are terms generally understood in the field to mean towards (proximal) or away (distal) from the user (e.g. physician) side of the apparatus. Thus, "proximal", "proximally", or "proximal to" means towards the user side and, therefore, away from the patient's side. Conversely, "distal", "distally", or "distal to" means towards the patient's side and, therefore, away from the user's side.

The present invention relates to an injector system for injection of a medical implant. The injector system comprises a holding element having a proximal and distal end disposed with a chamber for containing the implant. The holding element is disposed with an exit port at the distal end for slidable ejection of the implant. The injector system further comprises a compliant member assembly comprising at least one compliant member configured to frictionally engage the implant to impede passage past or through compliant member assembly. The compliant member assembly may be further configured to prevent entry by the implant therein under a force of gravity e.g. when the injector system is in transit. The compliant member assembly is disposed in fixed relation to the holding element. The implant is slidable relative to the compliant member assembly once engaged within the compliant member assembly. The invention maintains the implant in the holding element during transport and storage of the injection system. In addition it maintains the integrity of the implant after injection. The compliant member allows the implant to remain intact when passing through the compliant member assembly. Accordingly, the reliability of the delivery (dose delivered and duration of the release) is ensured.

The injection system is suitable for injection of an implant into a body of a subject, preferably a mammalian (e.g. animal), preferably a human subject. In particular, it is suited for subcutaneous injection, intramuscular or intradermal injection of an implant.

The medical implant, also referred to herein as implant may be any suitable for injection. It typically has a solid state *i.e.* not a liquid nor a gas. It may be essentially incompressible or non-deformable. By non-deformable, it is meant that the implant retains its shape and size under the application of force, in particular, under the application of forces experienced during injection. The implant may be dimensioned for implantation subcutaneously, intramuscularly, or intradermally. It preferably has an essentially cylindrical shape, though other shapes are envisaged such as those having oval, C-shaped, or polygonal profile. It may have rounded or flat ends. It is preferably longitudinal. A longitudinal direction of the implant may be generally aligned with longitudinal directions of the holding element chamber, and needle bore. It may be made from a bio-compatible material. The medical implant has a general profile that is a transverse cross section *i.e.* a section perpendicular to a longitudinal axis of the implant. Where the medical implant is cylindrical, the general profile is circular.

The implant may be provided with one or more active pharmaceutical ingredients. The implant may be configured for the slow release of one or more active pharmaceutical ingredients. An active pharmaceutical ingredient may be, for instance, an LHRH agonist or antagonist. An active pharmaceutical ingredient may be Buserelin, Triptorelin, Leuprorelin, Goserelin, Deslorelin, Histerlin, Avorelin, Nafarelin, Lutrelin, Cystorelin, Gonadorelin, Detirelix, or Luliberin. The implant may be provided with a combination of one or more of the aforementioned active pharmaceutical ingredients The implant may be biodegradable. The implant may be formed essentially from a biodegradable polymeric material, such as poly(α-esters), polyurethane, poly(ester amide), poly(ortho esters), polyanhydrides, polyphosphoester.

The implant may comprise an electronic device. It may comprise one or more electronic sensors, one or more radio-frequency transmitters, one or more radio-frequency receivers. The electronic components may be encapsulated in a non- biodegradable polymeric material.

The implant may be radio-opaque and act as a medical imaging marker.

The holding element is disposed with a chamber for containing the implant. The holding element has a body, typically formed from a rigid material such as polycarbonate or polypropylene, styrenics polymer (ABS-acrylonitrile butadiene styrene), ABS, cyclic olefin copolymer, polyethylene or polyolefin plastics. The body may be longitudinal. The body may be essentially cylindrical or frustoconical. The holding element has a proximal and distal end. The holding element has a chamber dimensioned for containing the implant. The chamber is preferably dimensioned to contain the entire implant. The chamber is preferably dimensioned to contain an entire axial length of the implant. The chamber is used to store the implant so that the injector system can be supplied to the user ready for use. The holding element may not form a part of the needle assembly.

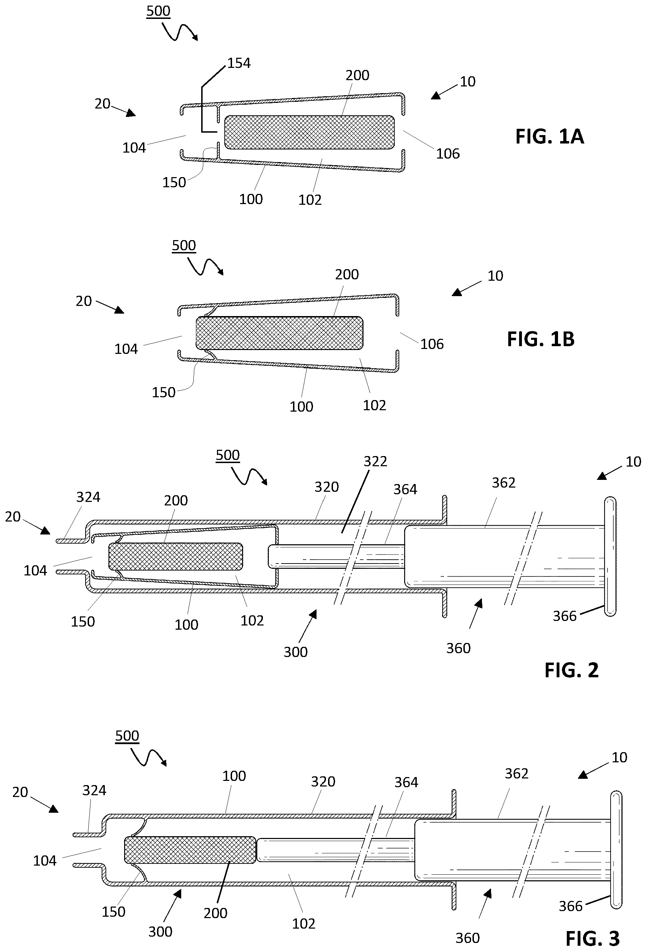

The holding element may be disposed within a syringe barrel as shown, for instance in **FIG. 2****.** The holding element may be attached in fixed relation to the barrel. The holding element may be formed from a syringe barrel as shown in **FIG. 3****.** The syringe may be any type of syringe of the art. For instance, a standard manual syringe, or an automatic syringe as described, for instance in WO 2014/174519. The syringe may incorporate a needle protection mechanism to shield the needle after injection of the implant as described, for instance in EP 0 966 983 or EP 1 235 603 and which are generally known in the art.

The distal end of the holding element is disposed with an exit port for passage of the implant. The exit port is an opening in the body of the holding element, disposed at the distal end, typically the distal terminal end. The exit port is configured for slidable ejection of the implant therethrough. Accordingly the exit port is larger than the maximum profile of the implant. The exit port is smaller than a transverse cross-sectional profile of the holding element chamber. The system, more preferably the holding element is configured such that the exit port can communicate with a needle assembly. In particular the exit port aligns with a bore of a needle such that the implant passing through the exit port enters the needle bore. A central axis of the exit port may be co-axial with a central axis of a needle bore.

The holding element may comprise a coupling for attachment to a needle assembly. Where the holding element is contained within a barrel of a syringe, the syringe tip (e.g. **FIG. 2**, 324) couples with a needle assembly. Where the holding element is a barrel of a syringe, the syringe tip (*e.g.* **FIG. 3**, 324) couples with a needle assembly.

The proximal end of the holding element is disposed with an entry port for passage of a plunger deployment rod. The entry port is an opening in the body of the holding element, disposed at the proximal end, typically the proximal terminal end. The plunger deployment rod is slidable relative to the entry port, and displaces the implant by the application of an axial force. More specifically the plunger deployment rod is configured to apply an axial force to the implant for ejection from the chamber. The entry port is larger than the maximum profile of the plunger deployment rod.

The needle assembly may be disposed distal of the holding element. The needle assembly is attached to or attached with respect to the holding element such that the implant can pass from the chamber and through the needle bore upon deployment. The attachment in respect of the holding element may be direct attachment, or an indirect attachment, for instance, via one or more adapters. The needle assembly may be repeatably dismountable with respect to the holding element. It may be essentially permanently attached with respect to the holding element. A transverse cross-section of the bore may be smaller than a transverse cross-section of the holding element.

The needle assembly comprises a needle component for puncture of an organ, for instance, skin or muscle. The needle is typical of a syringe needle of the art, having a longitudinal form, sharpened at one end for puncture of the skin, and containing a bore connecting the distal end of the needle component to the proximal end of the needle component. When attached to the holding element, connection is made between exit port and the proximal end of the bore of the needle component. The needle bore is dimensioned for the passage for the implant. The needle is configured for a subcutaneous, intramuscular, or intradermal injection route.

The needle assembly may further comprise a coupling component (e.g. hub) for attachment of the needle component to or with respect to the holding element. The attachment to or with respect to the holding element refers to a direct attachment, or to an indirect attachment, for instance, via one or more adapters or via a syringe barrel in which the holding element is placed (e.g. FIGs. 2 or 3). The coupling component is typically a push-fit connector.

The system further comprises a compliant member assembly, comprising one or more compliant members, configured to receive the implant. The compliant member assembly is configured for the passage of the implant therethrough. The compliant member assembly has a through-passage connecting a distal side of the compliant member assembly to its proximal side. The compliant member assembly (150) has a biased (resting) state in which the at least two compliant members (152) additively form a partial or total occlusion of the through-passage. The compliant member assembly acts as a barrier between the chamber and the exit port. It is configured to frictionally engage the implant to impede passage therethrough. The compliant member assembly retains the implant at least partially within the holding element. The injector system preferably contains only one compliant member assembly.

The compliant member assembly is configured to frictionally engage the implant thereby impeding passage therethrough. Preferably, the compliant member assembly frictionally engages with the implant when the implant passes into and through the through-passage under an injection force. By frictionally engaged, it is meant that the compliant member assembly applies a frictional force to the implant as seen for instance in **FIG. 1B****.** The force is applied in a radial direction. With frictionally engagement of the implant, each compliant member deforms by an axial movement and the size of the occlusion is reduced. When the implant is engaged with the compliant member, the latter acts as a brake to impede the passage of the implant relative to the compliant member assembly. By impeding it is meant increasing the amount of force required to advance the implant compared with when there is no engagement. The impeding preferably locks the position of the implant relative to the compliant member assembly when mutually engaged, which lock may be overcome by a force greater than gravity, preferably by a certain injection force *i.e.* a force applied axially to the implant. The injection force may be equal to or less than 10N, for instance less than or equal to 6N. The injection force may be greater than 3N.

Typically, frictional engagement arises during injection. The compliant member assembly is configured to retain the implant at least partially within the holding element by apply a retaining or frictional force to the implant.

The compliant member may be further configured as a compliant mechanical stop or mechanical stop. It prevents passage of the implant through the compliant member assembly under gravity. This stop functionality arises during transport of the injector system containing the implant, typically when the implant is not engaged with the compliant member assembly as shown, for instance, in **FIG. 1A**, and prevents the injector from falling out from the chamber. Accordingly, the compliant member assembly may be configured as a mechanical stop to retain the implant within the holding element. Typically the compliant members of the compliant member assembly are biased such that they at least partially occlude the exit port of the holding element. The implant may abut against the compliant member assembly without engaging with it. The mechanical stop function can be overcome by an axial force applied to the implant for instance by a syringe plunger deployment rod. With the application of sufficient force to the implant, the implant engages with the compliant member assembly; the compliant member assembly applies a breaking force to the implant.

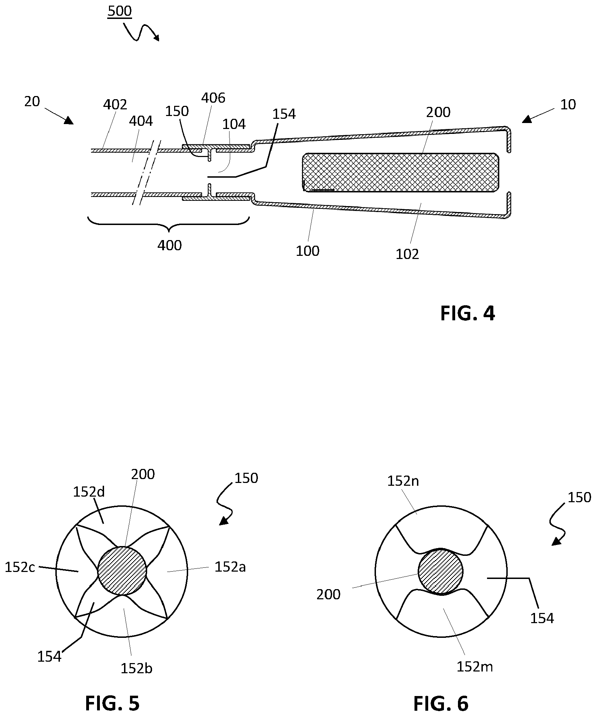

The compliant member assembly may be fixed in relation to holding element (as shown for instance in **FIG. 1**) and/or to the needle assembly (as shown for instance in **FIG. 4**). The compliant member assembly may be fixed in relation to or attached to the wall of the holding element chamber. The compliant member assembly may be fixed in distal relation to the needle bore. The compliant member assembly may be fixed in relation or attached to an inner wall of the needle assembly coupling component (as shown for instance in **FIG. 4**).

The compliant member assembly may contain at least one compliant member biased to block passage of the implant therethrough (*e.g.* **FIGs. 5** and **6**). The compliant member assembly may contain at least two compliant members. As is understood in the art, a compliant member is member that can be deformed by application of an external mechanical force, and returns to its original shape (biased or resting state) when the force is released. A compliant member may be a leaf spring. In the biased (resting) state (i.e. without the application of force), the compliant member assembly may have an essentially planar configuration. The planar configuration may be perpendicular to a central axis of the injector system, holding element or needle assembly coupling component. Upon the application of force by the implant, the at least one compliant member yields, and the distal end of the implant is able to advance past the compliant member, so engaging with it. The force of the compliant member against the implant when mutually engaged applies a braking friction force to the implant that impedes its passage through the compliant member.

The compliant member assembly may comprise 1, 2, 3 or 4 or more compliant members, preferably 2, 3 or 4 compliant members. Each compliant member may have a similar shape and/or size. Two or more compliant members may have a similar shape and/or size. One or more compliant member may have a different shape and/or size from the remainder of the compliant members. Each compliant member projects, preferably radially (optionally perpendicularly ± 10 deg), towards a central axis of the injector system, holding element or needle assembly coupling component. Each compliant member project perpendicularly ± 10 deg, to a central axis of the injector system, holding element or needle assembly coupling component. The compliant members may be arranged in the compliant member assembly to form an essentially circular profile i.e. outer shape. The compliant members are circular or annular segments, preferably of the same size and shape, arranged as segments within a circle or annular ring. The 1, 2, 3 or 4 or more compliant members in the biased state may additively form a partial or total occlusion of the through-passage. By additively, it is meant that the size or footprint of each compliant member additively combines to form the extent of the occlusion. The compliant member assembly may comprise an aperture defined by the compliant members, preferably by inward pointing edges of the compliant members, having a minimum width (e.g. diameter) smaller than the minimum width (e.g. diameter) of the implant. The minimum width of the implant is measure across a transverse cross section i.e. a section perpendicular to a longitudinal axis of the implant.

The compliant members may be attached at the circle or annular ring periphery to the holding element, or to the needle assembly, or to the coupling component. The segmented compliant members may be attached at the circle or annular ring periphery to the wall of the holding element chamber, preferably towards the distal end, or to the wall of the needle bore, or more preferably incorporated into a coupling component of the needle assembly.

The compliant member assembly is configured to align the implant with the exit port and with the bore of the needle component. For instance, it may essentially co-axially align a longitudinal axis of the implant with a central axis of the exit port. The compliant member assembly may be configured to align the implant with the bore of the needle. For instance, it may essentially co-axially align a longitudinal axis of the implant with a longitudinal axis of the needle bore. Alignment may be achieved, for instance, by disposing the compliant members so that forces applied by the compliant members act in a net radial direction on the implant to align it with a longitudinal axis of the needle bore and/or with the central axis of the exit port. For instance, the compliant members may be arranged evenly around a periphery or circumference of the bore, and/or be diametrically or symmetrically opposed. A compliant member may be made from any suitable compliant material *i.e.* a material that has an inherent spring-like quality or that can adopt a spring-like form. Examples of suitable materials include polypropylene, silicone rubber, nitinol, polyethylene (PE), thermoplastic elastomer (TPE), polyacetal (POM).

Advantageously, the invention improves the reliability of the injector system, preventing unwarranted loss of the implant prior during storage or transport. It has been found that the use of a compliant member assembly does not increase the injection force required by the user compared with a standard injector. The injection force reflects the amount of effort needed by the user to advance the implant though the holding element chamber and needle. Further, the invention reduces or prevents deformation of the implant. The compliant member allows the implant to remain intact when passing through the compliant member assembly. Accordingly, the reliability of the delivery (dose delivered and duration of the release) is improved. Compared with non-compliant ribs, the injector of the invention reduces injection force, and significantly reduces the injection forces needed for larger implant sizes.

By aligning the implant with the needle bore, the compliant member assembly allows a decrease in the dimensional tolerance of the implant. In other words, the same injector system can be used with an implant manufactured with less-stringent dimensional uniformity, in particular in the transverse cross-section. By moving the location of the retaining mechanism (compliant member assembly) out from the needle bore *i.e.* distal of it, the size of the needle can be reduced, thereby reducing trauma and pain during injection.

For some applications, the injector system including the implant may be required to be supplied in a sterile state. The dimensions of the implant in particular of the transverse cross-section can be altered (e.g. size expansion or contraction) by the sterilisation process. Because the injector system contains one or more compliant members, these dimensional changes to the implant can be accommodated, and without a significant increase in injection force. Hence, the implant having a starting dimension will not fall out of the injector system before sterilisation and will still be able to pass through the compliant member assembly having expanded after sterilisation. The method for sterilization of maybe any, for example heat, gaseous technique or gamma irradiation. The gamma irradiation is preferred.

For other applications, different sizes of implant transverse profile may be available depending on the length of release time of an active pharmaceutical agent; the same injector system may be used for different implant sizes with a similar centering and retaining functionality, and without a significant increase in injection force.

**FIGs. 1A** and **B** show a cross-sectional view of a holding element **100** of an injector system **500** as described herein. The holding element **100** has a frustoconical body in which a chamber **102** is disposed, said holding element **100** having a proximal end **10** and a distal end **20.** The chamber **102** holds a medical implant **200,** typically for subcutaneous, intramuscular, and/or intradermal injection. An exit port **104** is provided at the distal end **20** of the holding element **100** for passage of the medical implant during deployment. An entry port **106** is provided at the proximal end **10** of the holding element **100** for passage of a deployment rod (not shown). Attached to the chamber **102** wall is a compliant member assembly **150.** The compliant member assembly **150** acts as a barrier between the chamber **102** and the exit port **104.** The compliant member assembly **150** has a through-passage (154) that is partially occluded. In **FIG 1A**, the compliant member assembly **150** acts as a barrier retaining the implant **200** under the force of gravity in the chamber **102**, in particular proximal **10** of the compliant member assembly **150.** In **FIG 1B**, the implant **200** has been advanced in a distal direction **20**, and engages with the compliant member assembly **150**, retaining it at least partially in the chamber **102.** The implant **200** passes through the partially through-passage (154), reducing the extent of occlusion.

**FIG. 2** shows the holding element **100** of **FIGs. 1A** and **1B** disposed within a barrel **320** of a syringe **300.** The syringe barrel **320** contains a holding chamber **322** for the holding element **100.** The syringe **300** further comprises a plunger **360** comprising a shaft **362**, a deployment rod **364** and a plunger head **366**, and the syringe barrel **320** terminates in a tip **324** that is a coupling for attachment to a needle assembly. The plunger **360** is in sliding co-operation with the syringe barrel **320,** and the deployment rod **364** attached to a distal **20** end of the shaft **362** is configured to contact the implant **200** and advance it through the needle bore. The distal **20** end of the barrel **320** is disposed with a tip **324** for attachment to a needle assembly. The exit port **104** of the holding element **100** is aligned with the syringe tip **324** such that the implant **200** can pass through both and into the needle bore.

**FIG. 3** shows a holding element **100** that is a barrel **320** of a syringe **300.** The syringe barrel **320** contains a chamber **102** for the implant **200.** The syringe **300** further comprises a plunger **360** having a shaft **362**, a deployment rod **364** and a plunger head **366**, and the syringe barrel **320** terminates in a tip **324** that is a coupling for attachment to a needle assembly. The plunger **360** is in sliding co-operation with the syringe barrel **320**, and the deployment rod **364** attached to a distal **20** end of the shaft **362** is configured to contact the implant **200** and advance it through the needle bore. Attached to the chamber **102** wall is a compliant member assembly **150.** The implant **200** has been advanced in a distal direction **20**, and is engaged with the compliant member assembly **150**, retaining it at least partially in the chamber **102.** The distal **20** end of the barrel **320** is disposed with a tip **324** for attachment to a needle assembly. The exit port **104** of the holding element **100** is aligned with the syringe tip **324** such that the implant **200** can pass through both and into the needle bore.

**FIG. 4** depicts a cross-sectional view a holding element **100** similar to that shown in **FIG. 1** coupled to a needle assembly **400.** The needle assembly **400** comprises a needle component **402** having a bore **404** which needle component **402** is attached at its proximal **10** end to the holding element **100** via a coupling component **406.** The compliant member assembly **150** is disposed within the coupling component **406** at the distal **20** end of the exit port **104.**

**FIG. 5** is a plan view of a compliant member assembly **150**, comprising 4 segment leaflets that are compliant members **152a** to **152d** arranged in diametrically opposing pairs, wherein the implant **200** is engaged with the leaflets.

**FIG. 6** is a plan view of a compliant member assembly **150**, comprising 2 segment leaflet that are compliant members **152m** to **152n** arranged mutually diametrically opposing, wherein the implant **200** is engaged with the leaflets.

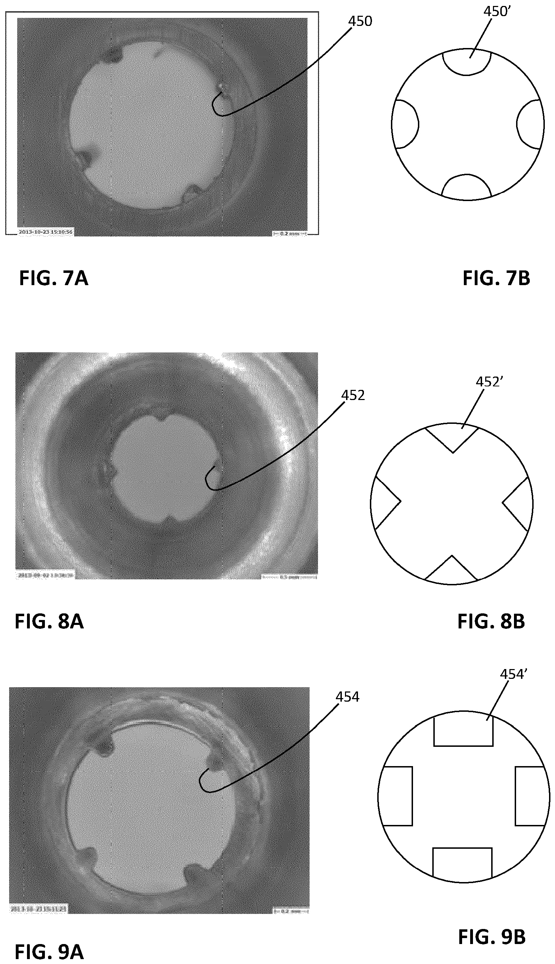

**FIG. 7A** is a photograph of an arrangement of four rigid ribs (**450**) each having a round profile; **FIG. 7B** is a schematic view of the configuration (**450'**).

**FIG. 8A** is a photograph of an arrangement of four rigid ribs (**452**) each having a triangular profile; **FIG. 8B** is a schematic view of the configuration (**452'**).

**FIG. 9A** is a photograph of an arrangement of four rigid ribs (**454**) each having a rectangular profile; **FIG. 9B** is a schematic view of the configuration (**454**').

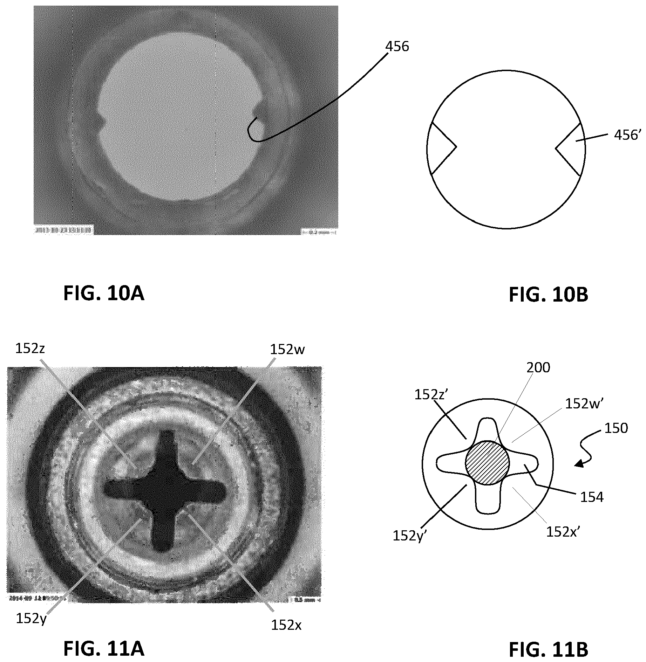

**FIG. 10A** is a photograph of an arrangement of two rigid ribs (**456**) each having a triangular profile; **FIG. 10B** is a schematic view of the configuration (**456**').

**FIG. 11A** is a photograph of a compliant member assembly according to the invention comprising four compliant members **152w-z**; **FIG. 11B** is a schematic view of the compliant member assembly of **FIG. 11A**, the four compliant members indicated (**152w**' to **z**') engaged with the implant **200**.

### Experimental

### Experiment 1

An injector system for injecting an implant into a subject (e.g. subcutaneously, intramuscularly, or intradermally) was prepared comprising a holding element contained inside a syringe barrel, the holding element containing the implant, the system being provided without the compliant member assembly. Eleven such systems were prepared, and each system was to be tested in trials for implant injection effort. The length and diameter of implants were comparable with each other and suitable for placement in the injection system. The length of each of the implants was 1.3 cm, the diameter of each of the implants was 0.118 cm. Each implant was made of polymer containing an active pharmaceutical ingredient.

For each of the injector systems without the compliant member assembly, the implant dropped out from the injector at the beginning of the trial or when installing the injector systems on the apparatus for testing. Three implants were lost during trial. On the eight implants remaining, seven had a diameter smaller than the lower tolerance limit and passed through the needle under the force of gravity. The last remaining sample was at minimum limit of tolerance and dropped out of the injector upon contact with the shaft. The test protocol described in Experiment 2 was hence not completed due to loss of the implant.

### Experiment 2

An injector system for injecting an implant into a subject (e.g. subcutaneously, intramuscularly, or intradermally) was prepared comprising a holding element contained inside a syringe barrel, the holding element containing the implant, the system being provided without the compliant member assembly. The injector system was provided with one or more rigid ribs to prevent unwarranted exit of the implant. The ribs were disposed in the needle assembly hub. Four different designs of ribs (A to D) were used as portrayed in **FIG. 7** to **10** respectively showing the rib profiles. The length and diameter of implants were comparable with each other and suitable for placement in the injection system.

The length of each of the implants was 1.3 cm, the diameter of each of the implants was 0.118 cm. Each implant was made of polymer containing an active pharmaceutical ingredient. The length of each rib was 0.02 cm in the direction of the central axis of the needle. The ribs were arranged to contact the implant; the maximum passage diameter through the ribs was less than the minimum diameter of the implant. Five such systems were prepared, and each system was tested in trials for implant injection effort. Injection effort was measured as follows: The injection system was placed vertically, with needle downwards, on a testing machine equipped with a clamping mechanism to retain the injection system in a fixed position. The syringe was advanced at a speed of 100 mm/min, and the maximum effort (force) applied over time to fully eject the implant was measured. The results are given in the Table 1 below. The ribs damage the implant after injection or the force needed to eject the implant is very high.

**Table 1 Results on testing of the properties of 4 different designs of injector systems (A to D); five copies of each design (#1 to #5) were tested**

| Property | **Design A** **FIG. 7A** & **B** | **Design B** **FIG. 8A** & **B** | **Design C** **FIG. 9A** & **B** | **Design D** **FIG. 10A & B** |
|---|---|---|---|---|
| | **Rounded rib profile x4** | **Triangular rib profile x4** | **Rectangular rib profile x4** | **Triangular rib profile x2** |
| **Injection force** | #1: 3.4 N | #1: 4.6 N | #1: 6.1 N | #1: 1.5 N |
| | #2: 4.0 N | #2: 3.6 N | #2: 6.8 N | #2: 1.4 N |
| | #3: 3.4 N | #3: 3.4 N | #3: 6.16 N | #3: 1.0 N |
| | #4: 4.0 N | #4: - | #4: 6.4 N | #4: 1.4 N |
| | #5: 3.6 N | #5: 3.9 N | #5: 7.7 N | #5: 1.6 N |
| **Comments** | No loss of implant | No loss of implant | No loss of implant | No loss of implant |
| | Reduction of implant profile | Reduction of implant profile | No effect on profile High force | High manufacturing tolerance required |

### Experiment 3

An injector system for injecting an implant into a subject (e.g. subcutaneously, intramuscularly, and/or intradermally) was prepared comprising a holding element contained inside a syringe barrel, the holding element containing the implant, the system being provided with the compliant member assembly of the invention. The compliant member assembly was disposed in the needle assembly hub. Exemplary design of compliant member assembly is shown in **FIGs. 11A** and **B**. One hundred and twenty copies of the injector system were made, sixty designed for use with an implant having a first (smaller) size (dimensions 1.3 cm length, 0.118 cm diameter), and sixty designed for use with an implant having a second (larger) size (dimensions 1.8 cm length, 0.147 cm diameter). Each implant was made of polymer containing an active pharmaceutical ingredient. The second-sized implant may be used to elute the active pharmaceutical ingredient over a longer time span. The implant injector systems used to inject the first- and second-sized implants each had an identical compliant member assembly. Each injector system was tested in trials for implant injection effort prior to and after sterilisation by irradiation as shown in Table 2. Injection effort was measured as described above. The results are given in the Table 2 below.

**Table 2 Results of testing properties of 2 injector systems having different sized implants using the same dimension of compliant member assembly, before and after sterilisation by irradiation.**

| | **Injector system 1: first-size implant** | | **Injector system 2: second-size implant** | |
|---|---|---|---|---|
| | **Before irradiation** | **After irradiation** | **Before irradiation** | **After irradiation** |
| **Number injector systems** | 30 | 30 | 30 | 30 |
| **Average force** | 2.08 N | 2.67 N | 3.32 N | 4.89 N |
| **Standard deviation** | 0.45 N | 0.6 N | 0.64 N | 0.74 N |
| **Comments** | No implant fell out from the injector before the test | | No implant fell out from the injector before the test | |
| | No deformation of implant and no material removed from the implant | | No deformation of implant and no material removed from the implant | |
| | Injection force is not excessive | | Injection force is not excessive | |
| | Visual control of the compliant member assembly shows a deformation of the compliant members after injection but without any material removed from the compliant members | | Visual control of the compliant member assembly shows a deformation of the compliant members after injection but without any material removed from the compliant members | |

### Experiment 4

An injector system for injecting an implant into a subject (e.g. subcutaneously, intramuscularly, and/or intradermally) was prepared comprising a holding element contained inside a syringe barrel, the holding element containing the implant, the system being provided with the compliant member assembly of the invention. The compliant member assembly was disposed in the needle assembly hub. Exemplary design of compliant member assembly is shown in **FIGs. 11A** and **B**. Sixty such systems were prepared and adapted to one target diameter implant. In each injector system, the implant was replaced with a calibrated rod made from steel of diameter at the lower diameter limit (0.110 cm) allowed for this specified injector system. Each injector system was tested in trials for implant injection effort prior to and after sterilisation by irradiation. Injection effort was measured as described above. The results are given in the Table 3 below.

**Table 3 Results of testing properties of a total of 60 injector systems using a calibrated rod in place of an implant, before and after sterilisation by irradiation.**

| | **Injector system** | |
|---|---|---|
| | **Before irradiation** | **After irradiation** |
| **Number injector systems** | 30 | 30 |
| **Average force** | 1.61 N | 1.99 N |
| **Standard deviation** | 0.10 N | 0.10 N |
| **Comments** | No calibrated rod fell out from the injector before the test, compliant member assembly was still efficient | |
| | Injection force was above 1N | |
| | Visual control of the compliant member assembly showed a deformation of the compliant members after injection but without any material removed from the compliant members. | |

## Claims

1. An injector system (500) for injection by puncture of an organ, of a non-deformable medical implant (200) into a subject comprising:
- a holding element (100) having a proximal (10) and distal (20) end disposed with a chamber (102) for containing the implant (200),
- wherein the holding element (100) is disposed with an exit port (104) at the distal end (20) for slidable ejection of the implant (200) therethrough and into a bore (404) of a needle component (402) of an injection needle assembly (400),
- a compliant member assembly (150) having a through-passage (154), comprising at least two compliant members (152), the compliant member assembly (150) having a biased state in which the at least two compliant members (152) additively form a partial or total occlusion of the through-passage (154), wherein:
- the compliant member assembly (150) is configured in the biased state as a mechanical stop adapted to stop slidable entry of the implant (200) into the bore (404) under a force of gravity,
- the through-passage (154) is configured to receive slidably the implant under a force of injection in which the at least two compliant members (152) deform and frictionally engage the implant (200),
- the compliant members (152) are arranged as segments (152a, b, c, d, n, m) within a circle or annular ring in the compliant member assembly (150),
- the exit port (104) is smaller than a transverse cross-sectional profile of the holding element chamber (102),
- the compliant member assembly (150) is configured to align the implant with the exit port (104) and the bore of the needle component.

2. The injector system (500) according to claim 1, wherein the compliant member assembly (150) comprises an aperture defined by the compliant members (152), preferably by inward pointing edges of the compliant members (152), the aperture having a minimum width smaller than a minimum width of the implant (200) transverse profile.

3. The injector system (500) according to claim 1 or 2, wherein the compliant member assembly (150) is biased in an essentially planar configuration.

4. The injector system (500) according to any one of claims 1 to 3, configured such that frictional engagement locks the position of the implant (200) relative to the compliant member assembly (150), wherein an injection force applied to the implant (200), preferably of less than 10N, overcomes the lock.

5. The injector system (500) according to any one of claims 1 to 4, further comprising a syringe (300) having a syringe barrel (320), wherein the holding element (100) is disposed within the syringe barrel (320).

6. The injector system (500) according to any one of claims 1 to 5, wherein the holding element (100) is a syringe barrel (320).

7. The injector system (500) according to any one of claims 1 to 6 further comprising the injection needle assembly (400) in fluid connection with the exit port (104) of the holding element (100), which needle assembly (400) comprises the needle component (402) for puncture of the organ of the subject disposed with a bore (404) for the passage of the implant (200) and further comprises a coupling component for attachment of the needle component to or with respect the holding element (100).

8. The injector system (500) according to any one of claims 1 to 7, wherein the compliant member assembly (150) is disposed within the holding element (100), or within a coupling component (406) of the needle assembly (400).

9. The injector system (500) according to any one of claims 1 to 8 further comprising the implant (200).

10. The injector system (500) according to any one of claims 1 to 9 configured for subcutaneous injection, intramuscular or intradermal injection of the implant (200).

11. The injector system (500) according to any one of claims 1 to 10, further comprising a needle protection mechanism.

## Patentansprüche

1. Injektorsystem (500) zur Injektion eines nicht verformbaren medizinischen Implantats (200) in ein Individuum durch Punktion eines Organs, umfassend:
- ein Halteelement (100), das ein proximales (10) und ein distales (20) Ende aufweist, das mit einer Kammer (102) zum Aufnehmen des Implantats (200) versehen ist,
- wobei das Halteelement (100) mit einer Austrittsöffnung (104) am distalen Ende (20) zum gleitenden Ausstoßen des Implantats (200) durch diese hindurch und in eine Bohrung (404) einer Nadelkomponente (402) einer Injektionsnadelbaugruppe (400) versehen ist,
- eine Baugruppe (150) nachgiebiger Elemente, die einen Durchgang (154) aufweist und mindestens zwei nachgiebige Elemente (152) umfasst, wobei die Baugruppe (150) nachgiebiger Elemente einen vorgespannten Zustand aufweist, in dem die mindestens zwei nachgiebigen Elemente (152) additiv eine teilweise oder vollständige Okklusion des Durchgangs (154) bilden, wobei:
- die Baugruppe (150) nachgiebiger Elemente in dem vorgespannten Zustand als ein mechanischer Anschlag ausgelegt ist, der dazu ausgebildet ist, ein gleitendes Eintreten des Implantats (200) in die Bohrung (404) unter einer Schwerkraft zu stoppen,
- der Durchgang (154) dazu ausgelegt ist, das Implantat unter einer Injektionskraft gleitfähig aufzunehmen, in der sich die mindestens zwei nachgiebigen Elemente (152) verformen und reibschlüssig mit dem Implantat (200) in Eingriff stehen,
- die nachgiebigen Elemente (152) als Segmente (152a, b, c, d, n, m) innerhalb eines Kreises oder Rings in der Baugruppe (150) nachgiebiger Elemente angeordnet sind,
- die Austrittsöffnung (104) kleiner als ein Querschnittsprofil der Halteelementkammer (102) ist,
- die Baugruppe (150) nachgiebiger Elemente dazu ausgelegt ist, das Implantat an der Austrittsöffnung (104) und der Bohrung der Nadelkomponente auszurichten.

2. Injektorsystem (500) nach Anspruch 1, wobei die Baugruppe (150) nachgiebiger Elemente eine Öffnung umfasst, die durch die nachgiebigen Elemente (152) definiert ist, vorzugsweise durch nach innen weisende Kanten der nachgiebigen Elemente (152), wobei die Öffnung eine minimale Breite aufweist, die kleiner als eine minimale Breite des Querprofils des Implantats (200) ist.

3. Injektorsystem (500) nach Anspruch 1 oder 2, wobei die Baugruppe (150) nachgiebiger Elemente in einer im Wesentlichen planaren Konfiguration vorgespannt ist.

4. Injektorsystem (500) nach einem der Ansprüche 1 bis 3, das derart ausgelegt ist, dass Reibungseingriff die Position des Implantats (200) relativ zu der Baugruppe (150) nachgiebiger Elemente arretiert, wobei eine auf das Implantat (200) angewandte Injektionskraft, vorzugsweise von weniger als 10N, die Arretierung überwindet.

5. Injektorsystem (500) nach einem der Ansprüche 1 bis 4, ferner umfassend eine Spritze (300), die einen Spritzenzylinder (320) aufweist, wobei das Halteelement (100) innerhalb des Spritzenzylinders (320) angeordnet ist.

6. Injektorsystem (500) nach einem der Ansprüche 1 bis 5, wobei das Halteelement (100) ein Spritzenzylinder (320) ist.

7. Injektorsystem (500) nach einem der Ansprüche 1 bis 6, ferner umfassend die Injektionsnadelbaugruppe (400) in Fluidverbindung mit der Austrittsöffnung (104) des Halteelements (100), wobei die Nadelbaugruppe (400) die Nadelkomponente (402) zur Punktion des Organs des Individuums umfasst, die mit einer Bohrung (404) für den Durchgang des Implantats (200) versehen ist, und ferner eine Kopplungskomponente zum Befestigen der Nadelkomponente an oder in Bezug auf das Halteelement (100) umfasst.

8. Injektorsystem (500) nach einem der Ansprüche 1 bis 7, wobei die Baugruppe (150) nachgiebiger Elemente innerhalb des Halteelements (100) oder innerhalb einer Kopplungskomponente (406) der Nadelbaugruppe (400) angeordnet ist.

9. Injektorsystem (500) nach einem der Ansprüche 1 bis 8, ferner umfassend das Implantat (200).

10. Injektorsystem (500) nach einem der Ansprüche 1 bis 9, das zur subkutanen Injektion, intramuskulären oder intradermalen Injektion des Implantats (200) ausgelegt ist.

11. Injektorsystem (500) nach einem der Ansprüche 1 bis 10, ferner umfassend einen Nadelschutzmechanismus.

## Revendications

1. Système d'injecteur (500) destiné à l'injection par ponction d'un organe, d'un implant médical indéformable (200) dans un sujet comprenant :
- un élément de maintien (100), présentant des extrémités proximale (10) et distale (20), pourvu d'une chambre (102) pour contenir l'implant (200),
- dans lequel l'élément de maintien (100) est pourvu d'un orifice de sortie (104) à l'extrémité distale (20) pour l'éjection coulissante de l'implant (200) à travers celui-ci et dans un alésage (404) d'un composant d'aiguille (402) d'un ensemble d'aiguille d'injection (400),
- un ensemble d'éléments élastiques (150) présentant un passage traversant (154), comprenant au moins deux éléments élastiques (152), l'ensemble d'éléments élastiques (150) présentant un état sollicité dans lequel les au moins deux éléments élastiques (152) forment de manière additive une occlusion partielle ou totale du passage traversant (154), dans lequel :
- l'ensemble d'éléments élastiques (150) est conçu à l'état sollicité en tant que butée mécanique adaptée pour arrêter l'entrée coulissante de l'implant (200) dans l'alésage (404) sous l'effet d'une force de gravité,
- le passage traversant (154) est conçu pour recevoir de manière coulissante l'implant sous l'effet d'une force d'injection dans laquelle les au moins deux éléments élastiques (152) se déforment et viennent en prise par frottement avec l'implant (200),
- les éléments élastiques (152) sont agencés en segments (152a, b, c, d, n, m) dans un cercle ou une bague annulaire dans l'ensemble d'éléments élastiques (150),
- l'orifice de sortie (104) est plus petit qu'un profil transversal en coupe de la chambre d'élément de maintien (102),
- l'ensemble d'éléments élastiques (150) est conçu pour aligner l'implant avec l'orifice de sortie (104) et l'alésage du composant d'aiguille.

2. Système d'injecteur (500) selon la revendication 1, dans lequel l'ensemble d'éléments élastiques (150) comprend une ouverture délimitée par les éléments élastiques (152), de préférence par des bords tournés vers l'intérieur des éléments élastiques (152), l'ouverture présentant une largeur minimale inférieure à une largeur minimale du profil transversal de l'implant (200).

3. Système d'injecteur (500) selon la revendication 1 ou 2, dans lequel l'ensemble d'éléments élastiques (150) est sollicité dans une configuration sensiblement plane.

4. Système d'injecteur (500) selon l'une quelconque des revendications 1 à 3, conçu de telle sorte qu'une prise par frottement verrouille la position de l'implant (200) par rapport à l'ensemble d'éléments élastiques (150), dans lequel une force d'injection appliquée à l'implant (200), de préférence inférieure à 10 N, permet de lever le verrouillage.

5. Système d'injecteur (500) selon l'une quelconque des revendications 1 à 4, comprenant en outre une seringue (300) présentant un cylindre de seringue (320), dans lequel l'élément de maintien (100) est disposé à l'intérieur du cylindre de seringue (320).

6. Système d'injecteur (500) selon l'une quelconque des revendications 1 à 5, dans lequel l'élément de maintien (100) est un cylindre de seringue (320).

7. Système d'injecteur (500) selon l'une quelconque des revendications 1 à 6, comprenant en outre l'ensemble d'aiguille d'injection (400) en liaison fluidique avec l'orifice de sortie (104) de l'élément de maintien (100), lequel ensemble d'aiguille (400) comprend le composant d'aiguille (402), pour la ponction de l'organe du sujet, pourvu d'un alésage (404) pour le passage de l'implant (200) et comprend en outre un composant de couplage pour la fixation du composant d'aiguille sur ou par rapport à l'élément de maintien (100).

8. Système d'injecteur (500) selon l'une quelconque des revendications 1 à 7, dans lequel l'ensemble d'éléments élastiques (150) est disposé à l'intérieur de l'élément de maintien (100), ou à l'intérieur d'un composant de couplage (406) de l'ensemble d'aiguille (400).

9. Système d'injecteur (500) selon l'une quelconque des revendications 1 à 8, comprenant en outre l'implant (200).

10. Système d'injecteur (500) selon l'une quelconque des revendications 1 à 9, conçu pour l'injection sous-cutanée, intramusculaire ou intradermique de l'implant (200).

11. Système d'injecteur (500) selon l'une quelconque des revendications 1 à 10, comprenant en outre un mécanisme de protection d'aiguille.
